# EUROPEAN PATENT APPLICATION

(11) **EP 1 887 017 A1**
(43) Date of publication of application: **13.02.2008**
(21) Application number: 06076550.0
(22) Date of filing: 09.08.2006
(51) Int. Cl.: C08B 37/00, C07H 3/04, C07H 3/06, A61K 31/7016, A61K 31/702, A23L 1/30, A23L 1/09, A61P 1/12, A61P 1/04

(54) **Prebiotic carbohydrate**

(71) Applicant: Friesland Brands B.V., 7943 PE Meppel (NL)
(72) Inventor: de Slegte, Jaap, 7006 KK Doetinchem (NL); Sinclair, Haydn Robert, Camerton, BA2 0PP Bath (GB); van den Heuvel, Elisabeth G. H. M., 3572 GS Utrecht (NL)
(74) Representative: van Loon, C.J.J.

(57) **Abstract**

The invention relates to the use of a carbohydrate represented by the formula Galₙ-Xₒ-Galₚ, wherein
n and p are integers in the range of 0-9, with the proviso that the sum of n and
p is in the range of 1-9,
o is an integer in the range of 1-9, with the proviso that the sum of n, o and p is 2-10,
Gal is a galactose moiety,
X₀ represents a monosaccharide moiety different from Gal (if o=1) - which monosaccharide moiety is optionally an acetylated or reduced monosacharide moiety - or an oligosaccharide moiety (if o>1), comprising at least one monosaccharide moiety different from Gal - which monosaccharide moiety is optionally an acetylated or reduced monosacharide moiety -
in the manufacture of a food or medicament for providing an effect in a distal part of the colon, in particular for stimulating beneficial bacteria in the distal part of the colon of a human, another mammal or another vertebrate.

## Description

The invention relates to a novel prebiotic carbohydrate, and especially a synthesized prebiotic carbohydrate to a composition comprising such carbohydrate, to a method for preparing a prebiotic carbohydrate and to the use of a prebiotic carbohydrate.

A prebiotic is a food ingredient that passes the small intestines without being substantially digested, such as a non-digestible oligosaccharide (NDO), and that beneficially affects the host by selectively stimulating the growth and/or activity of one or a limited number of potentially beneficial bacteria in the colon. For a food ingredient it must fulfil the following criteria:
1) it should pass the upper gastrointestinal tract essentially unhydrolysed and unabsorbed;
2) it should be selectively fermentable by one or a limited number of potentially beneficial bacteria in the colon, such as *Lactobacilli* and *Bifidobacteria,* which are stimulated to grow and/or become metabolically activated;
3) it should be able to alter the colonic intestinal flora towards a healthier composition by increasing, for example, the number of saccharolytic species while reducing the number of putrefactive micro-organisms.

One way to improve the colonic intestinal flora is to add potentially beneficial micro-organisms, for instance *Bifidobacteria,* to a food. Because such micro-organisms, in particular *Bifidobacteria,* are susceptible to oxygen and heat, their application in foods has been limited.

Therefore, there has been increased interest in including prebiotics in food. Prebiotics have shown effectiveness, usually endure normal food processing better than micro-organisms and may selectively target indigenous beneficial bacteria.

However, some prebiotics known in the art have shown a number of adverse effects. Previous experiments in rats have shown that the mucosal barrier of the colon may be damaged by some known NDO - such as lactulose and fructocarbohydrates (FOS) - due to a fast ('explosive') fermentation in the first (upper) part of the colon.

In addition, diarrhoea may develop if the intake of prebiotics exceeds the fermentation capacity of the colonic bacteria.

WO 2005/003329 A1 describes prebiotic galactocarbohydrates consisting of galactose residues and optionally a glucose residue. The described carbohydrates are mainly fermented in the proximal part of the colon, as appears from analyses of the colonic content of sacrificed animals (see "conclusion" at page 25 of WO 2005/003329 A1).

The present inventors conclude that formed metabolites will be absorbed rapidly and thus do not directly expose their protective effect on the distal part of the colon, such as a protective effect on distally oriented colon cancer.

WO 01/41581 describes an infant formula which may contain a substrate for micro-organisms in the intestinal flora. The substrate is added for creation and maintenance of a beneficial flora in the whole gut. As substrate lactose, lactulose and several other oligosaccharides are mentioned.

In recent years, it has become clear that several colonic diseases are caused by toxic metabolites produced by the intestinal flora. In particular, the inventors have realised that inflammatory diseases and cancer are prevalent in the distal part of the colon. It is thought that the intestinal flora in this region of the gut may play an important role in the onset or maintenance of such disorders. Dietary carbohydrates form the main fermentable substrate in the proximal colon; after this substrate is degraded by bacterial fermentation, protein takes over as the dominant fermentable substrate towards the distal areas. The products of bacterial protein metabolism include toxic and potentially carcinogenic compounds such as amines, ammonia and phenolic compounds. A correlation between colonic diseases and the fermentation of proteinaceous compounds is established.

Oligofructoses are also almost completely fermented in the proximal part of the large intestine, as reported in WO 01/60176. In WO 01/60176, it has been suggested that by using fructose polymers (inulins) the rate of fermentation may be decreased in the hope of allowing the compound to reach more distal parts of the colon. However agave inulin is reported to be as easily fermented as the lower molecular weight oligofructoses. It is suggested to combine special long-chain inulins that are hard to digest by bacteria in the colon in combination with short chain oligofructoses or inulins that are easy to digest by bacteria in the colon.

Carbohydrate polymers may be less applicable in food products due to their structural effects. In addition, polysaccharides tend to be less selective in the stimulation of bacterial flora.

It is an object of the invention to provide and especially synthesize a novel prebiotic carbohydrate, that can be used as an alternative to known prebiotics.

It is in particular an object to provide a prebiotic carbohydrate for selectively stimulating beneficial bacteria in a distal part of the colon.

It is a further object to modulate the short chain fatty acid production of bacteria found in a distal part of the colon quantitatively and/or qualitatively.

Yet a further object is to provide a use for a prebiotic directed at realising an effect in a distal part of the colon.

One or more objects which may be solved in accordance with the present invention will become clear from the remainder of the description.

It has now surprisingly been found that one or more of these objects are realised by providing a carbohydrate with a specific composition and preferably a relatively short chain length.

It is in particular surprising that it is possible that relatively short carbohydrates, administered to a subject, are effective in reaching the distal part of the colon and then are digested by beneficial bacteria, especially since it is taught in the art to increase the length of the prebiotic carbohydrates, in order to reduce digestion in the small intestines and proximal part of the colon.

Thus, the invention provides a number of uses of a specific class of carbohydrates directed at realising an effect in a distal part of the colon.

Accordingly, the invention relates to the use of a carbohydrate from the group represented by the formula Galₙ-Xₒ-Galₚ, wherein
n and p are integers in the range of 0-9, with the proviso that the sum of n and p is in the range of 1-9, preferably of 1-7, more preferably of 2-6;
o is an integer in the range of 1-9, preferably of 1-6, more preferably 1, 2 or 3, with the proviso that the sum of n, o and p is 2-10, preferably 2-8, more preferably 3-7;
Gal is a galactose moiety; and
X₀ represents a monosaccharide moiety different from Gal (if o=1) - which monosaccharide moiety is optionally an acetylated or reduced monosaccharide moiety - or X₀ represents an oligosaccharide moiety (if o>1), comprising at least one monosaccharide moiety different from Gal - which monosaccharide moiety is optionally an acetylated or reduced monosaccharide moiety - in the manufacture of a food or medicament for selectively stimulating beneficial bacteria in the distal part of the colon of a human, another mammal or another vertebrate. In particular, the invention may be used to preferentially stimulate the growth of *Bifidobacteria* and/or lactobacteria in the distal part of the colon.

Further, the invention relates to the use of a carbohydrate represented by said formula Galₙ-Xₒ-Galₚ
as a vehicle for delivering moiety X₀ to bacteria in the distal part of the colon of a human, another mammal or another vertebrate.

Further, the invention relates to the use of a carbohydrate represented by said formula Galₙ-Xₒ-Galₚ for modulating - in particular stimulating - the production of one or more short chain fatty acids - in particular at least one fatty acid selected from propionic acid and butyric acid - by bacteria found in a distal part of the colon.

In addition, the invention relates to the use of a carbohydrate represented by said formula Galₙ-Xₒ-Galₚ in the manufacture of a food or medicament for selectively reducing detrimental bacteria - in particular E. *coli* - in the distal part of the colon of a human, another mammal or another vertebrate.

Further, the invention relates to the use of a carbohydrate represented by said formula Galₙ-Xₒ-Galₚ in the manufacture of a food or medicament for the prophylactic or therapeutic treatment of a disorder selected from the group consisting of cancers, in particular a cancer manifesting in a distal part of the colon, more in particular colon cancer; intestinal disorders, in particular inflammatory bowel disease, ulcerative colitis, Crohn's disease, diarrhoea and constipation.

The invention further relates to a novel carbohydrate and a novel carbohydrate mixture, as will be described in further detail below.

The carbohydrate (used) according to the invention is preferably a synthetic carbohydrate.

The carbohydrate may be used in accordance with the invention in pure form, in a mixture of carbohydrates or in another composition, such as a food, a food supplement or a pharmaceutical composition.

A carbohydrate (used) according to the invention is usually not substantially digested in the small intestines. A carbohydrate used according to the invention has been found to have a relatively low fermentation rate under intestinal conditions. Accordingly, when ingested, usually at least a large fraction of the carbohydrate molecules reaches the colon, in particular a distal part of the colon. This allows preferential stimulation of beneficial bacteria, such as bifidobacteria and/or lactobacteria and/or reduction of putrative bacteria, such as E. *coli,* present in the distal part of the colon. The distal part is defined herein as the transversal and descending sections of the colon, in particular the descending section of the colon.

It is further contemplated that a carbohydrate (used) according to the invention is fermented relatively slowly in the colon, and thereby may offer a reduced risk of a detrimental effect such as damage to the mucosal barrier of the colon.

It is also contemplated that due to a prolonged fermentation in the colon, a carbohydrate (used) according to the invention has a higher threshold dose above which intolerance to the carbohydrate occurs. This allows administration of a higher dose which may result in higher health effects.

It is further contemplated that a carbohydrate (used) according to the invention contributes to a reduced fermentation of proteinaceous matter in the distal part of the colon, and thereby to a reduced formation of hazardous metabolites of the proteinaceous matter.

It has further been found that by varying the specific monosaccharide units (the monosaccharide unit(s) other than the galactose unit) of which the carbohydrate is composed, the activity of the bacteria can be selectively modulated. Such use is in particular suitable for modulating the production of short chain fatty acids, such as acetate, lactate, propionate and/or butyrate.

Preferably, at least one X is selected from monosaccharide moieties, including acetylated and reduced monosaccharide moieties, different from glucose (Glc) and fructose (Fru). More preferably at least an X bound to a Gal is different from Glc and Fru. For improved availability to microorganisms; distal part of the colon, the carbohydrate of the invention that is preferably enzymatically produced or synthesized usin β-galactosidase, is preferably not raffinose, stachyose or lactulose.

The nature of the monosaccharide units forming X₀ may be selected to achieve a specific effect, *e.g*. to stimulate the production of a specific organic acid or to realise a decrease or a reduction in the number of detrimental bacteria such as *E. coli* in the colon.

Xₒ may in particular comprise at least one moiety selected from the group consisting of pentose moieties, hexose moieties, deoxysugar moieties, N-acetylated sugar moieties, Glc-Fru, Gal-Glc and sugar alcohol moieties.

Preferably, Xₒ comprises at least one moiety selected from the group consisting of moieties of D-/L-arabinose, L-ribose, D-/L-xylose, D-lyxose, D-mannose, L-rhamnose, D-/L-fucose, N-acetylglucosamine, N-acetyl galactosamine, sucrose, lactulose, leucrose, raffinose, melizitose, stachyose, melibiose, cellobiose, maltose, isomaltose, maltotriose, trehalose, lactitol, D-xylitol, D-arabitol, D-mannitol and galactitol.

Several aspects of the carbohydrate may contribute to a specific effect of the carbohydrate on the bacteria.

D-galactose, D-glucose, N-acetylated glucosamine (GlcNAc), L-fucose and sialic acid are the main components of human milk oligosaccharides (HMO), with lactose at the reducing end of most HMO. Branching and chain elongation of HMO can be achieved by attachment of GlcNAc with a β1-3 or β1-6 bond to D-galactose, or by addition of lactosamines, L-fucose and sialic acid. Lacto-N-tetraose (Galβ1-3GlcNAcβ1-3Galβ1-4Glc) and lacto-N-fucopentaose (Fucα1-2Galβ1-3GlcNAcβ1-3Galβ1-4Glc) are the two most common HMO. HMO are indigestible and reach the colon of the infant where they can be utilised by the intestinal flora. Earlier studies reported that higher amounts of *Bifidobacteria* could be found in the faeces of breast-fed infants than in the faeces of formula-fed infants (Willis et al, Br Med J. 1973;13;67-72.; Bullen et al., (J Med Microbiol 1976;9:335-344). In recent studies, the effect of HMO on pathogenic bacteria has been investigated. *Escherichia coli* bind to mucosal receptors containing fucosylated oligosaccharides, sialyllactose and sialylgalactosides. *E. coli* adhere to these structures in the HMO when these are present instead of binding to the colonic wall. Similar mechanisms have been shown for *Helicobacter pylori* with sialyllactose and for *Pseudomonas aeruginosa* with Galβ1-3GlcNAc and Galβ1-4GlcNAc (reviewed by Kunz et al., Annu. Rev. Nutr. 2000;20:699-722).

A carbohydrate wherein X₀ provides one or more moieties selected from D-arabinose, D-xylose, D-mannose, D-rhamnose, D-ribose, L-arabinose, L-xylose, L-mannose, L-rhamnose and L-ribose, may be particularly useful for stimulating beneficial bacteria, in particular lactobacteria and/or *Bifidobacteria,* in the distal part of the colon and/or for reducing putrefactive microorganisms in the distal part of the colon.

In particular for stimulating the adhesion of beneficial bacteria to the colon in the distal part of the colon, a carbohydrate of the invention comprising a mannose unit is considered of use. This helps to increase colonisation of bacteria such as lactobacteria.

Also the degree of polymerisation may influence the way in which the beneficial bacteria are stimulated. By selecting relatively large or relatively short carbohydrates or by providing mixtures of different lengths different effects can be realised.

In particular for a low fermentation prior to the (distal part of) colon, it is preferred that the number of monosaccharide residues (n+o+p) is at least 3.

Further, it has been found that the chain length may have an effect on the production of fatty acids by the micro-organisms found in the colon.

In particular, a carbohydrate having a relatively long chain length or a mixture predominantly comprising carbohydrates with a relatively long chain length (about 5 to about 8 monosaccharide units), such as a penta-, hexa-, hepta- or octosaccharides, may considerably alter the production of short chain fatty acids such as lactate, acetate, propionate and/or butyrate by beneficial bacteria, in particular the propionate and/or butyrate production.

In particular such D-mannogalactans, L-rhamnogalactans, L-xylogalactans and D-arabinogalactans have been found to increase propionate production. Moreover, D-mannogalactans and L-rhamnogalactans have been found to increase butyrate production.

The carbohydrate may also be used to decrease the amount of *E. coli* bacteria. Relatively short carbohydrates or mixtures with predominantly relatively short chain lengths, in particular a tri-, tetra- or pentasaccharide or a mixture comprising predominantly tri- to pentasaccharides, have been found particularly suitable for this purpose. Especially such L-rhamnogalactan and L-arabinogallactan have been found to be very effective for this purpose.

The type of linkage between the monosaccharide moieties may affect the digestion properties in the smaller intestines and/or the colon. Preferably at least one of the bonds between the monosaccharide units is a β-linkage - such as β1-1, β1-2, β1-3, β1-4 or β1-6 linkage. For an increased degree of indigestibility more preferably at least one of the bonds is a β1-1, β1-2, β1-3, or β1-6 linkage. For making the monosaccharide unit represented by "X" available to a large extent to bacteria in a distal part of the colon, the carbohydrate preferably at least comprises a β1-1, β1-2, β1-3, or β1-6 bond between a Gal and an X.

The invention further relates to a novel carbohydrate mixture, which may in particular be suitable for any one or more of the uses of the invention. A carbohydrate mixture of the invention comprises at least two different carbohydrates represented by the formula Galₙ-Xₒ-Galₚ, wherein
n and p are integers in the range of 0-9, with the proviso that the sum of n and p is in the range of 1-9,
o is an integer in the range of 1-9, with the proviso that the sum of n, o and p is 2-10,
Gal is a galactose moiety,
X₀ represents a monosaccharide moiety different from Gal (if o=1) - which monosaccharide moiety is optionally an acetylated or reduced monosaccharide moiety - or an oligosaccharide moiety (if o>1), comprising at least one monosaccharide moiety different from Gal - which monosaccharide moiety is optionally an acetylated or reduced monosaccharide moiety.

Preferably, the carbohydrate mixture comprises
- 0-45 wt. %, in particular 10-40 wt. %, more in particular 20-35 wt. % of at least one disaccharide of the formula Galₙ-Xₒ-Galₚ;
- 0-50 wt. %, preferably 10-45 wt. %, more preferably 20-40 wt. % of at least one trisaccharide of the formula Galₙ-Xₒ-Galₚ;
- 0-50 wt. %, preferably 5-45 wt. %, more preferably 10-40 wt. % of at least one tetrasaccharide of the formula Galₙ-Xₒ-Galₚ;
- 0-30 wt. %, preferably 1-25 wt. %, more preferably 2-10 wt. % of at least one pentasaccharide of the formula Galₙ-Xₒ-Galₚ;
- 0-30 wt. %, preferably 1-25 wt. %, more preferably 2-10 wt. % of at least one hexasaccharide of the formula Galₙ-Xₒ-Galₚ;
- 0-30 wt. %, preferably 1-25 wt. %, more preferably 2-10 wt. % of at least one heptasaccharide of the formula Galₙ-Xₒ-Galₚ;
- 0-30 wt. %, preferably 1-25 wt. %, more preferably 2-10 wt. % of at least one carbohydrate selected from the group consisting of octosaccharides, nonasaccharides and decasaccharides of the formula Galₙ-Xₒ-Galₚ
wherein all weight percentages are based on the total weight of the mixture. Herein at least two of the above carbohydrates are present. Preferences for n, o, p and X are as identified above.

The amount of said carbohydrates is usually 50-100 wt. %, preferably 90-100 wt. %, more preferably 95-100 wt. %, based on the total weight of the mixture. The balance is usually substantially formed by one or more other carbohydrates, for instance unreacted carbohydrates used as a starting material for preparing the oligosaccharides in the mixture and/or one or more carbohydrates selected from monosaccharides, and oligosaccharides in general.

The invention further relates to a carbohydrate that may in particular be suitable for any one or more of the uses of the invention. Such carbohydrate is represented by the formula Galₙ-Xₒ-Galₚ, wherein
n and p are integers in the range of 0-9, with the proviso that the sum of n and p is in the range of 1-9,
o is an integer in the range of 1-9, with the proviso that the sum of n, o and p is 2-10, preferably 3-10, more preferably 3-7,
Gal is a galactose moiety,
Xₒ comprises at least one moiety selected from the group consisting of pentose moieties, hexose moieties, deoxysugar moieties, N-acetylated sugar moieties, oligosaccharide moieties comprising oligosaccharides comprising Glc-Fru and at least one other monosaccharide, oligosaccharide moieties comprising Gal-Glc and at least one other monosaccharide and sugar alcohol moieties. Preferred moieties X₀ are as identified above, when discussing possible uses and effects of a carbohydrate represented by the formula Galn-Xₒ-Galₚ.

A carbohydrate or carbohydrate mixture (used) according to the invention is preferably synthesised. Synthesising is considered advantageous over isolation (if the carbohydrate or carbohydrate mixture is found in a natural source) or hydrolysis from a polysaccharide. It is contemplated that synthesis allows easier and/or better control of the purity of the carbohydrate respectively easier and/or better control of the purity and/or composition or carbohydrate mixture.

The carbohydrate or carbohydrate mixture (used) according to the invention can be prepared by enzymatically catalyzed transgalactosylation using beta-galactosidase, and especially retaining beta-galactosidase; preferably a beta-galactosidase of *bacillus circulans* is used. The galactose function of lactose is transferred onto the acceptor substrate 'X' resulting in the formation of the carbohydrate of the invention. The carbohydrate or carbohydrate mixture may *e.g*. be prepared based on a method generally described in F. van Rantwijk, M. Woudenberg-van Oosterom and R. A. Sheldon (1999). Glycosidase-catalysed synthesis of alkyl glycosides. Journal of Molecular Catalysis B: Enzymatic 6, 511-532. Further reference is made to P.F. Ekhart & E. Timmermans: Bulletin of the IDF 313, pages 59-64.

In accordance with the invention it is generally not necessary to encapsulate the carbohydrate(s) used according to the invention, in order to reach a distal part of the colon without substantially having been degraded (non-fermented). In particular good results have been achieved with non-encapsulated carbohydrates wherein the sum of n, o and p is at least 3.

If desired, the carbohydrates may be present in a capsule or in the form of coated particles. The coating/capsule is usually chosen such that it substantially remains undegraded until it reaches the colon. Suitable encapsulation/coating techniques are known in the art, e.g., from WO 00/74501 A1 and WO 02/085415 A1. In particular, carbohydrates which are fermented relatively fast (in the colon), such as lactose, are preferably encapsulated or coated.

The invention further relates to a food composition comprising a carbohydrate mixture or a carbohydrate of the invention. A food composition usually further comprises at least one other component suitable for use in a food. In principle it can be any GRAS (generally regarded as safe) component, in particular any edible compound.

Preferred food compositions include compositions selected from the group consisting of infant formulas, foods for the elderly, foods for diabetics, cereals, dough-based products, beverages and dairy products.

In an infant formulation of the invention, the amount of the carbohydrate (used) according to the invention, preferably is in the range of 0.3-0.6 g/100 ml formula or based on dry weight: 2-5 wt. %.

The invention further relates to a pharmaceutical composition comprising a carbohydrate mixture or a carbohydrate of the invention. Regarding a pharmaceutical purpose, a carbohydrate according to the invention may in particular be used (in the manufacture of a medicament) for treating a disorder of the gastro-intestinal tract. Preferred pharmaceutical compositions include compositions for treating cancer, in particular colon cancer, and compositions for treating a gut disorder, in particular inflammatory bowel disease. Preferably the composition is enterally administered. The daily dose preferably is in the range of 1-20 g of the carbohydrate or carbohydrate mixture per day, more preferably 1 to 5 g/day, in particular for teenagers and for adults.

The invention will now be illustrated by the following examples.

### Example 1 synthesis

Novel galactans were synthesised enzymatically by the transfer of galactose from a lactose molecule to an acceptor substrate or another lactose. After synthesis the galactans were fractionated to remove mono-saccharides and lactose and to obtain high or medium chain fractions or a mixture of both (only for D- and L-arabinogalactans and L-xylogalactans).

For the synthesis of arabino and xylogalactans a solution of lactose (0.7 M) and d-arabinose, 1-arabinose or 1-xylose (0.55 M) is prepared in demineralized water at 50°C and pH 6.5. The enzyme β-galactosidase of *Bacillus circulans* was added to a concentration of 20 lactose-units per gram carbohydrate. After 5 hours, the reaction is terminated by heating the reaction mixture at 80°C for 10 minutes. Non-reacted lactose is hydrolysed at 40°C for 4 hours using Maxilact L5000® (DSM), an enzyme specific for the hydrolysis of lactose (100 units/gram carbohydrate). After this reaction, the mixture is autoclaved for 15 minutes at 120°C and filtered. The final reaction product was analysed using HPAEC-PAD. The degrees of polymerisation were determined using LC-MS.

### Example 2 effect on organic acids production

The galactans were tested in non-pH controlled faecal batch culture experiments as follows. Each tube contained 9 mL anaerobic medium (pH 7) containing per liter: peptone 0.78 g, NaCl 0.49 g, Na₂HPO₄.12H₂O 7.1 g, KH₂PO₄1.6 g, Bile salts no.3 0.4 g, Cysteine.HCl 0.5 g, K₂HPO₄.3H₂O 0.5 g, MgSO₄ 0.01 g, CaCl₂.2H₂O 0.007 g, Hemin-solution (0.5 g/L) 10 mL, Tween 80 2 mL, Vitamin K1 10 µL, Resazurin-solution 4 mL. To each tube, 0.5 mL faecal slurry and 0.5 mL carbohydrate-PBS solution (10 % w/v) were added. Healthy adults who had not used antibiotics for at least three months donated stool samples. Within 30 min following defaecation, faecal slurries were prepared from 1 part freshly voided faecal sample and 9 parts pre-reduced phosphate-buffered saline (PBS) pH 7 (10 % w/v). The fermentations were carried out in triplicate, using faecal slurries from three different donors per experiment. Pure PBS and a glucose-PBS-solution were treated in the same way and used as negative and positive controls, respectively. Incubation and sample collection were carried out in the anaerobic cabinet (90 % N₂, 5 % CO2, 5 % H₂) at 37 °C. The experiments were conducted over a period of 24-78 hours.

The galactocarbohydrate Vivinal-GOS® (V-GOS) was used as a reference.

Recovery was measured using DIONEX HPLC, with a PA1 (Ion exchange) column and a PAD detection (pulsed amperometric detection). Fermentation of medium length carbohydrates (mixtures of predominantly tri-, tetra- and pentasaccharides) show a release of the acceptor substrate and lactose and other disaccharides after 24 hrs of fermentation. The presence of the mono-saccharides after 24h suggests a two-stage fermentation of the new galactans in the colon. This was confirmed by the analyses of organic acids, measured using a high performance liquid chromatography (HPLC) method with an ion-exchange column (HPX-87H), 0.006 M H₂SO₄ as eluent, and UV-detection at 205 nm. For carbohydrates containing a mannose residue this is illustrated in Figure 1, showing short chain fatty acid (SCFA) production (including lactate) in unstirred faecal batch cultures over 78 hrs. All values are means of triplicates.

In particular, D-mannogalactans and D-arabinogalactans showed a considerable increase between 8-78 h in organic acids as compared to V-GOS.

Besides a quantitative difference for several carbohydrate mixtures qualitative difference were observed.

The term "high" means mixtures predominantly comprising carbohydrates having 5-7 monomeric units and "medium" means mixtures predominantly comprising carbohydrates having 3-5 monomeric units.

For instance, compared to V-GOS, the organic acid ratio formed after 24h of fermentation of the new galactans showed the following differences:
D-mannogalactan mixture "high" and L-rhamnogalactan mixture "high" showed a higher proportion of acetate, propionate and butyrate, and a lower proportion of lactate.
L-xylogalactan mixture "high" showed a higher proportion of acetate and propionate and a lower proportion of lactate.
D-arabinogalactan mixture "high" showed a higher acetate and propionate proportion, but a lower proportion of butyrate and lactate.

### Example 3 intestinal flora changes

The intestinal flora changes in the cultures of Example 2 were determined using QPCR. For the isolation of bacterial DNA from faecal samples, the QIAamp DNA Stool Mini Kit from QIAgen was used. The primers and probes for the detection of bifidobacteria, lactobacilli, *E. coli,* some clostridia (*C. perfringens, C. paraputrificum, C. tertium* and *C. butyricum*), and *Bacteroides* were based on 16S rDNA gene sequences, retrieved by the website http://greengenes.lbl.gov. From these sequences forward primers, reverse primers and TaqMan probes were designed using the Primer Express software. To check specificity, the selected primers and probes were compared to all available 16S rDNA gene sequences using the BLAST database search program. Applied Biosystems manufactured primers and probes.

It was found that the increase in bifidobacteria was comparable to the increase in the cultures treated with V-GOS. This was in particular the case for D-mannogalactan mixture "high", D-mannogalactans "medium", L-rhamnosegalactan mixture "high", L-rhamnogalactan mixture "medium", D-arabinogalactan mixture "high", D-arabinogalactans mixture "medium", L-arabinogalactan mixture "high", L-arabinogalactan mixture "medium", both sucrogalactan mixtures "high" and "medium", and both FOS-galactan mixture "high" and "medium".

For lactobacilli, carbohydrates of the invention showed an improved stimulation, compared to V-GOS. Although after 8 hours the increase in lactobacilli tended to be less, after 24 hours the increase tended to be more than for V-GOS. This is an indication that the fermentation prolonged, allowing the carbohydrate to migrate into the distal part of the colon and be available for bacteria in the distal part of the colon.

This effect was in particular found for D-mannogalactan mixture "medium", L-rhamnogalactan, L-xylogalactan, L-arabinogalactan (both "medium" and "high"), D-arabinogalactan mixture containing predominantly di- to octosaccharides,. sucrogalactan mixture "medium", FOS galactan mixture "high", and FOS galactan mixture "medium".

The effect of the carbohydrates of the invention on the detrimental *E. coli* bacteria was also determined.

For V-GOS the number of *E. coli* was about the same after 8 hours and after 24 hours. However in the cultures containing D-mannogalactan mixture "high", D-mannogalactan mixture "medium", L-rhamnogalactan mixture "high", L-rhamnogalactan mixture "medium", L-arabinogalactan mixture "high", L-arabinogalactan mix containing predominantly di- to octosaccharides, both sucrogalactan mixtures "high" and "medium", and both FOS-galactan mixtures "high" and "medium" a decrease in *E. coli* bacteria count was observed between 8 hours and 24 hours. The largest effect was observed with the cultures comprising L-rhamnogalactan mixture "medium", L-arabinogalactan mixture "medium", L-arabinogalactan mix containing predominantly di- to octosaccharides, and both sucrogalactan mixtures "high" and "medium".

## Claims

1. Use of a carbohydrate represented by the formula Galₙ-Xₒ-Galₚ,
wherein
n and p are integers in the range of 0-9, with the proviso that the sum of n and p is in the range of 1-9,
o is an integer in the range of 1-9, with the proviso that the sum of n, o and p is 2-10,
Gal is a galactose moiety,
X₀ represents a monosaccharide moiety different from Gal (if o=1) - which monosaccharide moiety is optionally an acetylated or reduced monosacharide moiety - or an oligosaccharide moiety (if o>1), comprising at least one monosaccharide moiety different from Gal - which monosaccharide moiety is optionally an acetylated or reduced monosacharide moiety -
in the manufacture of a food or medicament for selectively stimulating beneficial bacteria in the distal part of the colon of a human, another mammal or another vertebrate.

2. Use of a carbohydrate represented by the formula Galₙ-Xₒ-Galₚ,
wherein
n and p are integers in the range of 0-9, with the proviso that the sum of n and p is in the range of 1-9,
o is an integer in the range of 1-9, with the proviso that the sum of n, o and p is 2-10,
Gal is a galactose moiety,
X₀ represents a monosaccharide moiety different from Gal (if o=1) - which monosaccharide moiety is optionally an acetylated or reduced monosacharide moiety - or an oligosaccharide moiety (if o>1), comprising at least one monosaccharide moiety different from Gal - which monosaccharide moiety is optionally an acetylated or reduced monosaccharide moiety -
as a vehicle for delivering moiety X₀ to bacteria in the distal part of the colon of a human, another mammal or another vertebrate.

3. Use of a carbohydrate represented by the formula Galₙ-Xₒ-Galₚ,
wherein
n and p are integers in the range of 0-9, with the proviso that the sum of n and p is in the range of 1-9,
o is an integer in the range of 1-9, with the proviso that the sum of n, o and p is 2-10,
Gal is a galactose moiety,
X₀ represents a monosaccharide moiety different from Gal (if o=1) - which monosaccharide moiety is optionally an acetylated or reduced monosacharide moiety - or an oligosaccharide moiety (if o>1), comprising at least one monosaccharide moiety different from Gal - which monosaccharide moiety is optionally an acetylated or reduced monosaccharide moiety -
for modulating the production of one or more short chain fatty acids by bacteria found in a distal part of the colon.

4. Use of a carbohydrate represented by the formula Galₙ-Xₒ-Galₚ,
wherein
n and p are integers in the range of 0-9, with the proviso that the sum of n and p is in the range of 1-9,
o is an integer in the range of 1-9, with the proviso that the sum of n, o and p is 2-10,
Gal is a galactose moiety,
X₀ represents a monosaccharide moiety different from Gal (if o=1) - which monosaccharide moiety is optionally an acetylated or reduced monosacharide moiety - or an oligosaccharide moiety (if o>1), comprising at least one monosaccharide moiety different from Gal - which monosaccharide moiety is optionally an acetylated or reduced monosaccharide moiety -
in the manufacture of a food or medicament for selectively reducing detrimental bacteria in the distal part of the colon of a human, another mammal or another vertebrate.

5. Use of a carbohydrate represented by the formula Galₙ-Xₒ-Galₚ,
wherein
n and p are integers in the range of 0-9, with the proviso that the sum of n and p is in the range of 1-9,
o is an integer in the range of 1-9, with the proviso that the sum of n, o and p is 2-10,
Gal is a galactose moiety,
X₀ represents a monosaccharide moiety different from Gal (if o=1) - which monosaccharide moiety is optionally an acetylated or reduced monosaccharide moiety - or an oligosaccharide moiety (if o>1), comprising at least one monosaccharide moiety different from Gal - which monosaccharide moiety is optionally an acetylated or reduced monosacharide moiety -
in the manufacture of a food or medicament for the treatment of a disorder selected from the group consisting of cancers, in particular a cancer manifesting in a distal part of the colon; and intestinal disorders, in particular inflammatory bowel disease, ulcerative colitis, Crohn's disease, diarrhoea and constipation.

6. Use according to any one of the preceding claims, wherein
n and p are in the range of 0-6, with the proviso that the sum of n and p is in the range of 1-6, preferably in the range of 2-6
o is the range of 1-6, preferably 1, 2 or 3
with the proviso that the sum of n, o and p is 2-7, preferably 3-7.

7. Use according to any one of the preceding claims, wherein Xₒ comprises at least one moiety selected from the group consisting of pentose moieties, hexose moieties, deoxysugar moieties, N-acetylated sugar moieties, oligosaccharide moieties comprising Glc-Fru, oligosaccharide moieties comprising Gal-Glc and sugar alcohol moieties.

8. Use according to claim 7, wherein Xₒ comprises at least one moiety selected from the group consisting of moieties of D-/L-arabinose, L-ribose, D-/L-xylose, D-lyxose, D-mannose, L-rhamnose, D-/L-fucose, N-acetylglucosamine, N-acetyl galactosamine, sucrose, lactulose, leucrose, raffinose, melizitose, stachyose, melibiose, cellobiose, maltose, isomaltose, maltotriose, trehalose, lactitol, D-xylitol, D-arabitol, D-mannitol and galactitol.

9. Carbohydrate mixture comprising at least two different carbohydrates represented by the formula Galₙ-Xₒ-Galₚ, wherein
n and p are integers in the range of 0-9, with the proviso that the sum of n and p is in the range of 1-9,
o is an integer in the range of 1-9, with the proviso that the sum of n, o and p is 2-10,
Gal is a galactose moiety,
X₀ represents a monosaccharide moiety different from Gal (if o=1) - which monosaccharide moiety is optionally an acetylated or reduced monosacharide moiety - or an oligosaccharide moiety (if o>1), comprising at least one monosaccharide moiety different from Gal) - which monosaccharide moiety is optionally an acetylated or reduced monosacharide moiety.

10. Carbohydrate mixture according to claim 9, comprising
- 0-45 wt. %, in particular 10-40 wt. %, more in particular 20-35 wt. % of at least one disaccharide of the formula Galₙ-Xₒ-Galₚ;
- 0-50 wt. %, preferably 10-45 wt. %, more preferably 20-40 wt. % of at least one trisaccharide of the formula Galₙ-Xₒ-Galₚ ;
- 0-50 wt. %, preferably 5-45 wt. %, more preferably 10-40 wt. % of at least one tetrasaccharide of the formula Galₙ-Xₒ-Galₚ;
- 0-30 wt. %, preferably 1-25 wt. %, more preferably 2-10 wt. % of at least one pentasaccharide of the formula Galₙ-Xₒ-Galₚ;
- 0-30 wt. %, preferably 1-25 wt. %, more preferably 2-10 wt. % of at least one hexasaccharide of the formula Galₙ-Xₒ-Galₚ;
- 0-30 wt. %, preferably 1-25 wt. %, more preferably 2-10 wt. % of at least one heptasaccharide of the formula Galₙ-Xₒ-Galₚ;
- 0-30 wt. %, preferably 1-25 wt. %, more preferably 2-10 wt. % of at least one carbohydrate selected from the group consisting of octasaccharides, nonasaccharides and decasaccharides of the formula Galₙ-Xₒ-Galₚ ;
wherein all weight percentages are based on the total weight of the mixture.

11. Carbohydrate mixture according to claim 10, wherein the amount of said carbohydrates is 50-100 wt. %, preferably 90-100 wt. %, more preferably 95-100 wt. %, based on the total weight of the mixture.

12. Carbohydrate represented by the formula Galₙ-Xₒ-Galₚ, wherein
n and p are integers in the range of 0-9, with the proviso that the sum of n and p is in the range of 1-9,
o is an integer in the range of 1-9, with the proviso that the sum of n, o and p is 2-10,
Gal is a galactose moiety,
Xₒ comprises at least one moiety selected from the group consisting of pentose moieties, hexose moieties, deoxysugar moieties, N-acetylated sugar moieties, oligosaccharide moieties comprising oligosaccharides comprising Glc-Fru and at least one other monosaccharide, oligosaccharide moieties comprising Gal-Glc and at least one other monosaccharide and sugar alcohol moieties.

13. Carbohydrate according to claim 12, wherein the sum of n, o and p is 3-10, preferably 3-7.

14. Carbohydrate according to claim 12 or 13, wherein Xₒ is bound to at least one of the Gal moieties via a β1-1, β1-2, β1-3, β1-4, β1-5 or β1-6 linkage.

15. Carbohydrate according to any one of the claims 12-14, wherein Xₒ comprises at least one moiety selected from the group consisting of moieties of D-/L-arabinose, L-ribose, D-/L-xylose, D-lyxose, D-mannose, L-rhamnose, D-/L-fucose, N-acetylglucosamine, N-acetyl galactosamine, sucrose, lactulose, leucrose, raffinose, melizitose, stachyose, melibiose, cellobiose, maltose, isomaltose, maltotriose, trehalose, lactitol, D-xylitol, D-arabitol, D-mannitol and galactitol.

16. Carbohydrate according to claim 15, wherein Xₒ comprises at least one moiety selected from the group consisting of moieties of D-arabinose, D-xylose, D-mannose, D-rhamnose, D-melizitose, L-arabinose, L-xylose, L-mannose, L-rhamnose, L-ribose, L-melizitose, D-fucose and L-fucose.

17. Method for preparing a carbohydrate according to any one of the claims 12-16 or a carbohydrate mixture according to any one of the claims 8-10, comprising transferring a galactose moiety of lactose to a monosaccharide or oligosaccharide corresponding to the moiety Xₒ, in the presence of a β-galactosidase.

18. Food composition comprising a carbohydrate according to any one of the claims 12-16 or a mixture according to any one of the claims 8-10 and at least one additional food ingredient

19. Food composition according to claim 18, wherein the composition is selected from infant foods, foods for the elderly, foods for diabetics, cereals, dough-based products, beverages and dairy products.

20. Pharmaceutical composition comprising a carbohydrate according to any one of the claims 12-16 or a mixture according to any one of the claims 8-10 and at least one pharmaceutically acceptable carrier.
